# EUROPÄISCHE PATENTSCHRIFT

(11) **EP 1 239 956 B1**
(45) Veröffentlichungstag und Bekanntmachung des Hinweises auf die Patenterteilung: **27.07.2005**
(21) Anmeldenummer: 00985098.3
(22) Anmeldetag: 27.11.2000
(51) Int. Cl.: B01J 23/50, B01J 23/52, B01J 31/02, B01J 35/10, C07D 301/10

(54) **KATALYSATOREN AUF BASIS EDELMETALL UND TITAN-HALTIGER, ORGANISCH-ANORGANISCHER HYBRIDMATERIALIEN ZUR SELEKTIVEN OXIDATION VON KOHLENWASSERSTOFFEN**
CATALYSTS WHICH ARE BASED ON ORGANIC-INORGANIC HYBRID MATERIALS CONTAINING NOBLE METALS AND TITANIUM AND WHICH ARE USED FOR SELECTIVELY OXIDIZING HYDROCARBONS
CATALYSEURS A BASE DE MATERIAUX HYBRIDES ORGANO-INORGANIQUES CONTENANT DES METAUX PRECIEUX ET DU TITANE, UTILISES POUR L'OXYDATION SELECTIVE D'HYDROCARBURES

(30) Priorität: 09.12.1999 DE 19959525
(43) Veröffentlichungstag der Anmeldung: 18.09.2002
(73) Patentinhaber: Bayer MaterialScience AG, 51368 Leverkusen (DE)
(72) Erfinder: WEISBECK, Markus, 51067 Köln (DE); WEGENER, Gerhard, 40822 Mettmann (DE); WIESSMEIER, Georg, 51427 Bergisch Gladbach (DE); VOGTEL, Peter, 51373 Leverkusen (DE)
(86) Internationale Anmeldenummer: PCT/EP2000/011781
(87) Internationale Veröffentlichungsnummer: WO 2001/041921

(56) Entgegenhaltungen:
- EP-A- 1 005 907
- WO-A-00/64581
- WO-A-00/64582
- WO-A-00/74841
- WO-A-98/00413
- WO-A-99/26936
- WO-A-99/43431

## Beschreibung

Die vorliegende Erfindung betrifft Zusammensetzungen enthaltend Gold und/oder Silberpartikel und titanhaltige, organisch-anorganische Hybridmaterialien, wobei diese Hybridmaterialien Si-H-Gruppen auf die inneren und äußeren Oberfläche enthalten, ein Verfahren zu deren Herstellung sowie deren Verwendung als Katalysator zur selektiven Oxidation von Kohlenwasserstoffen. Die katalytisch aktiven Zusammensetzungen zeigen hohe Selektivitäten und Produktivitäten und sehr lange Katalysatorstandzeiten ohne Desaktivierung.

Die Direktoxidation von Ethen zu Ethenoxid durch molekularen Sauerstoff ist gut bekannt und wird kommerziell zur Produktion von Ethenoxid in der Gasphase angewendet. Der typische Katalysator für diese Anwendung enthält metallisches oder ionisches Silber, eventuell noch modifiziert mit verschiedenen Promotoren und Aktivatoren. Die meisten dieser Katalysatoren enthalten einen porösen, inerten Katalysatorträger mit geringen Oberflächen wie z.B. alpha-Aluminiumoxid, auf den Silber und Promotoren aufgebracht wurden. Ein Überblick zur Direktoxidation von Ethen in Gegenwart von geträgerten Silberkatalysatoren wurde von Sachtler et al. in Catalysis Reviews: Science and Engineering, 23 (1&2), 127-149 (1981) zusammengestellt.

In US-A-5 623 090 wird eine Gasphasen-Direktoxidation von Propen zu Propenoxid mit relativ kleinen Propenumsätzen (0,5-1 % Propenumsatz bez. auf eine 10 %ige Propen-Feedkonzentration), aber Propenoxid-Selektivitäten von > 90 % mit Sauerstoff als Oxidationsmittel beschrieben. Es handelt sich hierbei um eine Gold-Titandioxid-katalysierte Gasphasenoxidation mit molekularem Sauerstoff in Gegenwart von Wasserstoff bei Temperaturen von 40-70°C. Als Katalysator wird handelsübliches kristallines Titandioxid mit vorwiegend Anatasmodifikation (P 25, Degussa; 70 % Anatas u. 30 % Rutil) verwendet, das mit nanoskaligen Goldteilchen mit einer Abscheidung-Ausfällungsmethode (deposition-precipitation) belegt wird. Dieses Verfahren hat neben den relativ geringen Propenumsätzen den großen Nachteil, dass die offenbarten Katalysatoren mit der Zeit stark desaktivieren. Typische Halbwertszeiten bei Normaldruck und 50°C liegen bei 30-150 Minuten. Temperatur- und/oder Druckerhöhung zur Steigerung des Umsatzes verkürzen die Halbwertszeiten weiter.

Bekannt sind weiterhin rein anorganische Katalysatoren, bei denen Goldpartikel aus geeigneten löslichen Goldverbindungen durch deposition-precipitation auf einen Träger, bestehend aus dispergierten Titandioxidzentren auf einer rein anorganischen Siliziummatrix aufgebracht werden (WO-98/00415; WO-98/00414, EP-A1-0 827 779). WO-98/00415 und WO-98/00414 heben hervor, dass alle Titandioxidzentren amorph vorliegen, d. h., dass im Unterschied zu US-A-5 623 090 keine kristalline TiO₂-Phase vorliegt. Alle diese Katalysatoren, die durch Imprägnierung der rein anorganischen Siliziumdioxidoberfläche mit Titanprecursorn in Lösung und nachfolgender Goldbelegung durch Abscheidung-Ausfällung (deposition-precipitation) und anschließender Calcinierung unter Luftatmosphäre erhaltenen Materialien zeigen relativ geringe Propenumsätze, desaktivieren rasch (typische Halbwertszeiten liegen bei 10-50 h) und können deshalb nicht in großtechnischen Anlagen eingesetzt werden.

Aus WO-98/00413 sind Katalysatoren bekannt, bei denen Goldpartikel auf rein anorganische mikroporöse, kristalline Gerüstsilicate mit definierter Porenstruktur (z.B. TS-1, TS-2, Ti-Zeolithe wie Ti-Beta, Ti-ZSM-48 oder titanhaltige, mesoporöse Molekularsiebe wie z.B. Ti-MCM-41 oder Ti-HMS) aufgebracht werden. Alle diese rein anorganischen Gold-Silikalit-, bzw. Gold-Zeolith-Katalysatoren zeigen zwar gute Selektivitäten bei der partiellen Oxidation, die Umsätze der Kohlenwasserstoffe und vor allem die Katalysatorstandzeiten sind für die Anwendung in der chemischen Industrie aber völlig unzureichend.

Die beschriebenen Verfahren zur Katalysatorpräparation sind in Bezug auf Katalysatoraktivität und -standzeit höchst unzufriedenstellend. Für technische Prozesse, die mit wenig aktiven Katalysatoren arbeiten, werden riesige Reaktoren benötigt. Geringe Katalysatorstandzeiten bedingen Produktionsausfall während der Regenerierungsphase oder verlangen nach einem redundanten, kostenintensiven Produktionsweg. Es ist also eine Entwicklung von neuen Katalysatoren wünschenswert, die bei exzellenten Selektivitäten hohe Aktivitäten bei technisch interessanten Standzeiten erreichen können.

Es ist bekannt, organisch-anorganische Hybridmaterialien als Lack-Komponente einzusetzen (z.B. EP-A1-743 313). Die Kombination mit Edelmetallen und Titan oder der Einsatz als Katalysator ist unbekannt.

Die ältere Anmeldung DE 199 18 431.3 beschreibt eine geträgerte Zusammensetzung enthaltend Gold- und/oder Silberpartikel, Titanoxid und einen siliziumhaltigen Träger, die dadurch gekennzeichnet ist, dass die Zusammensetzung durch einen nachfolgenden Modifizierschritt an der Oberfläche Gruppen ausgewählt aus Siliziumalkyl-, Siliziumaryl, fluorhaltige Alkyl- oder fluorhaltige Arylgruppen trägt, sowie deren Verwendung als Katalysatoren zur Dirextoxidation von Kohlenwasserstoffen. Organisch-anorganische Hybridmaterialien als Träger werden nicht offenbart.

Die Anmeldung WO 99/43431 beschreibt Katalysatoren, bei denen Goldpartikel aus geeigneten löslichen Goldverbindungen durch deposition-precipitation auf einen Träger, bestehend aus dispergierten Titanoxidzentren auf einer rein anorganischen Siliziummatrix (durch Lösungsmittel-Imprägnierung mit Titanprecursorn) aufgebracht werden und nachträglich mit Silylierungsmittel oberflächenmodifiziert werden. Organisch-anorganische Hybridmaterialien als Träger werden nicht offenbart.

Die ältere Anmeldung DE 199 20 753.4 beschreibt eine geträgerte Zusammensetzung enthaltend Gold- und/oder Silberpartikel und ein amorphes Titan-Silizium-Mischoxid, dass dadurch gekennzeichnet ist, dass man das Titan-Siliziumhaltige-Mischoxid durch ein Sol-Gel-Verfahren herstellt. Es werden keine Zusammensetzungen, die Si-H-Gruppen tragen, offenbart.

Die ältere Anmeldung DE 199 25 926.7 beschreibt edelmetallhaltige Ti-Si-Mischoxide als Katalysatoren mit organisch-anorganischen Hybridmaterialien auf der Basis von Carbosiloxanen zur Dirextoxidation von Kohlenwasserstoffen, sowie deren Herstellung über den Sol-Gel-Prozeß. Jedoch werden keine Zusammensetzungen, die Si-H-Gruppen tragen, offenbart.

Eine Aufgabe der vorliegenden Erfindung bestand darin, neue Katalysatoren für technische Prozesse mit hohen Aktivitäten ohne Desaktivierung bei gleichzeitig exzellenten Selektivitäten bereitzustellen.

Eine weitere Aufgabe bestand darin, ein Verfahren zur Herstellung dieser Katalysatoren zu entwickeln.

Eine weitere Aufgabe bestand darin, ein technologisch einfaches Gasphasenverfahren zur selektiven Oxidation von Kohlenwasserstoffen mit einem gasförmigen Oxidationsmittel an diesen Katalysatoren zur Verfügung zu stellen, welches bei hohen Aktivitäten, sehr hohen Selektivitäten und technisch interessanten Katalysatorstandzeiten zu hohen Ausbeuten und geringen Kosten führt.

Eine weitere Aufgabe bestand darin, einen alternativen Katalysator zur Direktoxidation von Kohlenwasserstoffen bereitzustellen.

Eine weitere Aufgabe bestand darin, die Nachteile der bekannten Katalysatoren wenigstens teilweise zu beseitigen.

Die Aufgaben werden gelöst durch eine Zusammensetzung enthaltend Gold- und/oder Silberpartikel auf einem titanhaltigen, organisch-anorganischen Hybridmaterial, dadurch gekennzeichnet, dass die Zusammensetzung Si-H-Gruppen enthält.

Organisch-anorganische Hybridmaterialien im Sinne der Erfindung sind organisch modifizierte Gläser, die bevorzugt in Sol-Gel-Prozessen über Hydrolyse- und Kondensationsreaktionen meist niedermolekularer Verbindungen entstehen und im Netzwerk terminale und/oder verbrückende organische Gruppen enthalten.

Bevorzugt werden organisch-anorganische Hybridmaterialien mit großer spezifischer Oberfläche. Die spezifische Oberfläche sollte vorteilhaft mehr als 1 m²/g, bevorzugt im Bereich von 10-700 m²/g betragen.

Weiterhin werden organisch anorganische Hybridmaterialien mit modifizierter Oberfläche bevorzugt Modifizierte Oberfläche im Sinne der Erfindung bedeutet, dass der Anteil der Oberflächen-Silanolgruppen durch kovalente oder koordinative Anbindung von Gruppen ausgewählt aus Siliziumalkyl-, Siliziumaryl-, fluorhaltigen Alkyl- und/oder fluorhaltigen Arylgruppen verringert wurde.

Die erfindungsgemäße Zusammensetzung enthält Gold und/oder Silber auf dem organisch-anorganischen Hybridmaterial als katalytisch aktives Trägermaterial. In aktiven Katalysatoren liegt Gold und/oder Silber hauptsächlich als Elementpartikel (Analyse durch X-ray absorption spectroscopy) vor. Kleine Gold- und/oder Silberanteile können auch in einem höheren Oxidationszustand vorliegen. Nach TEM-Aufnahmen zu urteilen liegt der größte Anteil des vorhandenen Goldes und/oder Silbers auf der äußeren und inneren Oberfläche des Trägermaterials vor. Es handelt sich um Gold- und/oder Silbercluster im Nanometermaßstab. Bevorzugt besitzen die Goldpartikel einen Durchmesser im Bereich von 0,3 bis 50 nm, bevorzugt 0,9 bis 15 nm und besonders bevorzugt 0,9 bis 10 nm. Bevorzugt besitzen die Silberpartikel einen Durchmesser im Bereich von 0,5 bis 100 nm, bevorzugt 0,5 bis 40 nm und besonders bevorzugt 0,5 bis 20 nm.

Die Goldkonzentration sollte im Bereich von 0,001 bis 4 Gew.-%, bevorzugt 0,001 bis 2 Gew.-% und besonders bevorzugt von 0,005-1,5 Gew.-% Gold betragen.

Die Silberkonzentration sollte im Bereich von 0,005 bis 20 Gew.-%, bevorzugt 0,01 bis 15 Gew.-% und besonders bevorzugt von 0,02 bis 10 Gew.-% Silber betragen.

Aus ökonomischen Gründen sollte der Edelmetallgehalt die minimal notwendige Menge zur Erlangung höchster Katalysatoraktivität betragen.

Die Erzeugung der Edelmetallpartikel auf den Hybridmaterialien ist nicht auf eine Methode beschränkt. Zur Generierung von Gold- und/oder Silberpartikeln seien hier einige Beispielverfahren wie Abscheidung-Ausfällung (Deposition-Precipitation) wie in EP-B-0 709 360 auf S. 3, Z. 38 ff. beschrieben, Imprägnierung in Lösung, Incipient-wetness, Kolloid-Verfahren, Sputtern, CVD, PVD genannt. Es ist ebenfalls möglich, Vorläuferverbindungen der Edelmetalle direkt in den Sol-Gel-Prozeß zu integrieren. Nach Trocknung und Temperung der edelmetallhaltigen Gele werden ebenfalls nanoskalige Gold- und/ oder Silberpartikel erhalten. Unter Incipient-wetness wird hierbei die Zugabe einer Lösung enthaltend lösliche Gold- und/oder Silberverbindungen zum Trägermaterial verstanden, wobei das Volumen der Lösung auf dem Träger kleiner oder gleich als das Porenvolumen des Trägers ist. Somit bleibt der Träger makroskopisch trocken. Als Lösungsmittel für Incipient Wetness können alle Lösungsmittel verwendet werden, in denen die Edelmetallverläufer löslich sind, wie Wasser, Alkohole, Ether, Ketone, Ester, Acetale, halogenierte Kohlenwasserstoffe usw.

Bevorzugt werden nanoskalige Gold- und/oder Silberpartikel nach den Methoden Incipient Wetness und Lösungsmittel-Imprägnierung erzeugt.

Überraschend gelingt die Generierung von nanoskaligen Goldpartikeln aus löslichen Goldverbindungen wie Tetrachlorogoldsäure z. B. nach der Incipient-Wetness-Methode auch in Gegenwart von oligomeren oder polymeren Hilfsstoffen, wie Polyvinylpyrolidon, Polyvinylalkohol, Polypropylenglykol, Polyacrylsäure usw. oder in Gegenwart von komplexbildenden Komponenten wie Cyaniden, Acetylaceton, Ethylacetoacetat usw. Bevorzugt werden komplexbildenede Zusätze wie Cyanide, z.B. Alkali- oder Erdalkalicyanide, eingesetzt.

Die erfindungsgemäßen Zusammensetzungen können vorteilhaft vor und/oder nach der Edelmetallbelegung durch thermische Behandlung bei 100-1200°C in verschiedenen Atmosphären wie Luft, Stickstoff, Wasserstoff, Kohlenmonoxid, Kohlendioxid weiter aktiviert werden. Bevorzugt ist eine thermische Aktivierung bei 150-500°C in Sauerstoff oder in sauerstoffhaltigen Gasen wie Luft, oder Sauerstoff-Wasserstoff- bzw. Sauerstoff-Edelgas-Gemischen oder Kombinationen davon oder unter Inertgasen bei 150-1000°C wie Stickstoff und/oder Wasserstoff und/oder Edelgasen oder Kombinationen davon. Oft ist die Gegenwart von Kohlenmonoxid, Kohlendioxid und kleinen Mengen Wasser vorteilhaft. Besonders bevorzugt erfolgt die Aktivierung der erfindungsgemäßen Zusammensetzungen unter Inertgasen im Temperaturbereich von 200-600°C. Es kann aber auch vorteilhaft sein, die erfindungsgemäßen Trägermaterialien bei Temperaturen im Bereich von 200-1000°C zu tempern, diese mit Edelmetall zu belegen und anschließend erneut bei 200-600°C zu tempern.. Je nach gewählter Aktivierungstemperatur verändern chemische Prozesse die Struktur der erfindungsgemäßen Zusammensetzungen. So können die Zusammensetzungen nach der thermischen Behandlung z.B. Siliziumoxycarbideinheiten enthalten. Die thermisch aktivierten (getemperten) erfindungsgemäßen Zusammensetzungen zeigen häufig eine signifikant höhere katalytische Aktivität und eine verlängerte Standzeit im Vergleich zu bekannten Katalysatoren.

Die organisch-anorganischen Hybridmaterialien im Sinne der Erfindung enthalten bezogen auf Siliziumoxid zwischen 0,1 und 20 Mol-% Titan, bevorzugt zwischen 0,8 und 10 Mol-%, besonders bevorzugt zwischen 1,0 und 8 mol.%. Das Titan liegt in oxidischer Form vor und ist bevorzugt chemisch über Si-O-Ti-Bindungen homogen in das organisch-anorganische Hybridmaterial eingebaut oder angebunden. Aktive Katalysatoren dieser Art weisen nur sehr untergeordnet Ti-O-Ti-Domänen auf.

Ohne hieran gebunden sein zu wollen, nehmen wir an, dass in aktiven Katalysatoren Titan über Heterosiloxanbindungen an Silizium gebunden ist.

Neben Titan können die erfindungsgemäßen Zusammensetzungen weitere Fremdoxide, sogenannte Promotoren, aus der Gruppe 5 des Periodensystems nach IUPAC (1985), wie Vanadium, Niob und Tantal, bevorzugt Tantal, der Gruppe 3, bevorzugt Yttrium, der Gruppe 4, bevorzugt Zirkon, der Gruppe 8, bevorzugt Fe, der Gruppe 15, bevorzugt Antimon, der Gruppe 13, bevorzugt Aluminium, Bor, Thallium und Metalle der Gruppe 14, bevorzugt Germanium, enthalten

Diese Promotoren liegen vorteilhaft zum größten Teil homogen, d.h. mit relativ geringer Dömanenbildung, vor. Die eingebauten Promotoren "M" liegen in den organisch-anorganischen Hybridmaterialien in der Regel dispers vor. Die chemische Zusammensetzung dieser Materialien läßt sich über weite Bereiche variieren. Der Anteil des Promotorelementes liegt bezogen auf Siliziumoxid im Bereich von 0-10 Mol-%, bevorzugt bei 0-4 Mol-%. Selbstverständlich können auch mehrere verschiedene Promotoren eingesetzt werden. Die Promotoren werden bevorzugt in Form von im jeweiligen Lösungsmittel löslichen Promotor-Vorläuferverbindungen, wie Promotorsalzen und/oder Promotor-organischen Verbindungen, und/oder Promotororganisch-anorganischen Verbindungen eingesetzt.

Diese Promotoren können sowohl die katalytische Aktivität der Zusammensetzung als auch die Standzeit der Zusammensetzung bei katalytischen Oxidationsreaktionen von Kohlenwasserstoffen erhöhen.

Die erfindungsgemäßen Zusammensetzungen enthaltend Gold- und/oder Silberpartikel und Ti-haltige, organisch-anorganische Hybridmaterialien mit Si-H-Gruppen, können im getrockneten Zustand näherungsweise durch folgende empirische allgemeine Formel (I) beschrieben werden (die nach Modifizierung gebildeten Reste an der Oberfläche und gegebenenfalls unvollständig abreagierte Gruppen werden hier nicht berücksichtigt):

SiOₓ * Org * H * TiO_{y} * MO_{z} * E (I)

SiOₓ steht für Siliziumoxid, Org bedeutet in der Formel die, vorzugsweise im Sol-Gel-Prozeß aus den organisch-anorganischen Vorläufern gebildeten, nicht hydrolysierbaren organischen Bestandteile, H gibt den molaren Anteil an "Element"-H-Gruppen im Netzwerk an, wobei "Element" ungleich Kohlenstoff ist, M ist ein Promotor, bevorzugt Ta, Fe, Sb, V, Nb, Zr, Al, B, Tl, Y, Ge oder Kombinationen davon, E bedeutet Gold und/oder Silber (Edelmetall) und x, y und z stehen für die effektiv notwendige Anzahl an Sauerstoff, um die Valenzen von den organischanorganisch bzw. reinanorganischen Elementen Si, Ti, und M abzusättigen.

Die oben bezeichnete Zusammensetzung (1) läßt sich über weite Bereiche variieren.

Bezogen auf Siliziumoxid kann der Anteil von Org in Molprozent zwischen 0,01 und 200 % betragen. Bevorzugt liegt er zwischen 10 und 200 %, besonders bevorzugt zwischen 30 und 100 %. Der molare Anteil an Si-H-Einheiten bezogen auf Siliziumoxid kann zwischen 0,01 und 100 Mol-% variieren. Bevorzugt liegt der Anteil zwischen 0,05 und 80 %, besonders bevorzugt zwischen 0,1 und 50 Mol-%. Der Anteil von Titanoxid liegt bezogen auf Siliziumoxid zwischen 0,1 und 10 Mol-%, bevorzugt zwischen 0,5 und 8,0 %, besonders bevorzugt zwischen 0,5 und 7,0 %. Der Anteil von MO_{z} liegt bezogen auf Siliziumoxid zwischen 0 und 12 Mol-%. Der Anteil von E liegt bezogen auf die Gold- und/oder Silber-freie Zusammensetzung zwischen 0,001 und 15 Gew.-%. Bei Gold liegt er bevorzugt zwischen 0,001 und 2 Gew.-%, bei Silber bevorzugt zwischen 0,01 und 15 Gew.-%.

Die gestellten Aufgaben werden weiterhin gelöst durch ein Verfahren zur Herstellung der erfindungsgemäßen Zusammensetzungen enthaltend Gold- und/oder Silberpartikel und Ti-haltige, organisch-anorganische Hybridmaterialien mit Si-H-Gruppen.

Die Ti-haltigen, organisch-anorganischen Hybridmaterialien mit Silanwasserstoffeinheiten werden über Sol-Gel-Prozesse hergestellt. Dies geschieht beispielsweise durch Mischen geeigneter, meist niedermolekularer Verbindungen in einem Lösungsmittel, wonach durch Zugabe von Wasser und gegebenenfalls Katalysatoren (z.B. Säuren, Basen und/oder metallorganische Verbindungen und/oder Elektrolyten) die Hydrolyse- und Kondensationsreaktion eingeleitet wird. Die Durchführung solcher Sol-Gel-Prozesse ist dem Fachmann grundsätzlich bekannt.

Geeignete Vorläuferverbindungen für Silizium-, Titan- und Promotorzentren sind vorteilhaft entsprechende für den Sol-Gel-Prozess geeignete meist niedermolekulare organisch-anorganische Mischverbindungen oder eine Kombination aus entsprechenden anorganischen und organisch-anorganischen Mischverbindungen. Niedermolekular steht im Sinne der Erfindung für monomer oder oligomer. Bei ausreichender Löslichkeit sind auch polymere Vorläuferverbindungen von Silizium, Titan und Promotoren geeignet.

Der Sol-Gel-Prozeß basiert auf der Polykondensation hydrolysierter, kolloidal gelöster Metallkomponentengemische (Sol) unter Bildung eines Netzwerkes (Gel). Zur Verdeutlichung hierfür dient das nachfolgende Schema am Beispiel der Hydrolyse und Kondensation von Tetraalkoxysilanen zu einem rein anorganischen Polysiloxan-Netzwerk.

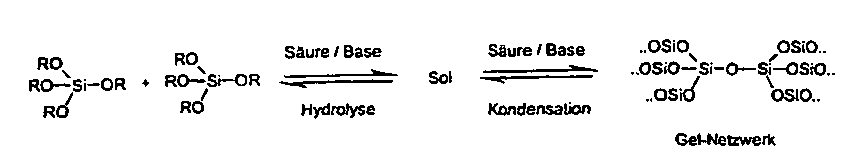

Die Hydrolyse erfolgt, indem hydrolysierbare Si- und/oder Ti-Vorläufer in einem geeigneten Lösungsmittel vorgelegt und dann mit Wasser gemischt werden und gegebenenfalls die Mischung mit Lösungsvermittler homogenisiert wird. Da die Hydrolyse von Siliziumvorläuferverbindungen unter Normalbedingungen langsam ist, benötigt man Katalysatoren, um sie schnell und vollständig ablaufen zu lassen (J. Livage et al., Chemistry of Advanced Materials: An Overview (Hrsg.: L.V. Interrante et al., VCH, New York, 1998, S. 389-448). Die entstehenden Silanole kondensieren unter Bildung von Siloxanverbindungen. Dabei entstehen gelöste Polysiloxan-Netzwerke. Verzweigung und Quervemetzung setzen sich fort, bis das Polymer so groß ist, dass der Gel-Übergang eintritt. Das Gel besteht zunächst aus einem festen Polymernetzwerk, das mit Lösungsmittel durchsetzt ist. Bei der anschließenden Trocknung schrumpft das Netzwerk unter Verlust des Lösungsmittels, wobei ein Xerogel aus Polysiloxan entsteht. Die Trocknung des Gels kann auch unter überkritischen Bedingungen erfolgen, dann bezeichnet man das entstehende Produkt als Aerogel (A. Baiker et al., Catal. Rev. Sci. Eng. 1995, 37, 515-556).

Bevorzugte Lösungsmittel für den Sol-Gel-Prozeß sind Alkohole wie tert.-Butanol, Isopropanol, Butanol, Ethanol, Methanol oder Ketone wie Aceton, und Ether wie z.B. THF oder tert.-Butylmethylether.

Geeignete Ausgangsmaterialien sind sämtliche dem Fachmann bekannten löslichen Silizium- und Titanverbindungen der allgemeinen Formel (II), die als Ausgangsmaterial für die entsprechenden Oxide oder Hydroxide dienen können,

[RₓM'(OR')₄₋ₓ] (II),

wobei
M' ausgewählt wird aus Silizium und Titan,
R und R' gleich oder verschieden sind und unabhängig voneinander ausgewählt werden aus der Gruppe C₁-C₁₂-Alkyl, und C₆-C₁₂-Aryl, wobei x = 0,1,2,3 ist und R' auch H sein kann.

Bei den organisch modifizierten Silanen sind eine oder mehrerer hydrolysierbarer Gruppen durch terminale und/oder verbrückte gesättigte (z. B. CH₃, C₂H₅, C₃H₇, ..) oder durch ungesättigte (z. B. C₂H₃, C₆H₅) R-Gruppe(n) ersetzt worden. Es können auch polyfunktionelle Organosilane, z. B. Silanole und Alkoxide, eingesetzt werden. Es können auch Silane, organisch modifiziert oder auch nicht, in Gegenwart von Di-oder Mehrfachalkoholen wie 1,4-Butandiol, zu organisch modifizierten Polysiloxanen umgesetzt werden. Verbrückte R-Gruppen (Alkylenreste) sind im Sinne der Erfindung verbrückte Strukturen wie kettenförmige, sternförmige (verzweigt), käfigförmige oder ringförmige Strukturelemente.

Die hier verwendeten modifizierten Silane unterscheiden sich deutlich von den üblicherweise verwendeten anorganischen Netzwerkbildner wie Alkoxysilanen [Si(OR)₄] mit vier hydrolysierbaren Gruppen, welche z.B. zur Herstellung von kristallinen Gerüstsilikaten mit definierter Porenstruktur (WO-98/00413; TS 1, TS 2, Ti-MCM 41 und 48) ausschließlich eingesetzt werden.

Im Gegensatz zu den erfindungsgemäßen Katalysatoren ist ein gemeinsames Merkmal aller zuvor bekannten Katalysatoren, dass Goldpartikel auf rein anorganischen Trägermaterialien, d. h. dass das Festkörpergerüst aus rein anorganischen Silizium-Sauerstoff- und Titan-Sauerstoff-Einheiten besteht, aufgebracht wurden.

Unter Alkyl werden sämtliche dem Fachmann bekannten terminalen und/oder verbrückten lineare oder verzweigte Alkylreste mit 1 bis 20 C-Atomen verstanden, wie Methyl, Ethyl, n-Propyl, i-Propyl, n-Butyl, i-Butyl, t-Butyl, n-Pentyl, i-Pentyl, neo-Pentyl, Hexyl und die weiteren Homologen, die ihrerseits wiederum substituiert sein können. Als Substituenten kommen hierbei Halogen, Nitro, oder auch Alkyl, Hydroxid oder Alkoxy, sowie Cycloalkyl oder Aryl in Frage, wie Benzoyl, Trimethylphenyl, Ethylphenyl, Chlormethyl, Chlorethyl und Nitromethyl. Bevorzugt werden unpolare Substituenten wie Methyl, Ethyl, n-Propyl, i-Propyl, n-Butyl, i-Butyl, t-Butyl und Benzoyl eingesetzt. Auch höhermolekulare und/oder oligomere organisch-anorganische Silizium- und Titanvorläufer sind geeignet, wie gamma-Glycidoxypropyltrimethoxysilan, 3,4-Epoxycyclohexyl-ethyl-trimethoxysilan, 1-(Triethoxysilyl)-2-(diethoxymethylsilyl)ethan, tris(gamma)-Trimethoxypropyl)-siliziumisocyanurat, peralkylierte Cyclosiloxane wie Hexamethylcyclotrisiloxan, Octamethyltetrasiloxan oder Decamethylpentasiloxan. Auch Polyalkyl(aryl)siloxane wie Polydimethylsiloxan sind geeignet.

Unter Aryl werden sämtliche dem Fachmann bekannte ein- oder mehrkernige Arylreste mit 6 bis 12 C-Atomen verstanden, wie Phenyl, Naphthyl oder Fluorenyl, die ihrerseits wiederum substituiert sein können. Als Substituenten kommen hierbei Halogen, Nitro oder auch Alkyl oder Alkoxyl, sowie Cycloalkyl oder Aryl in Frage, wie Bromphenyl, Chlorphenyl, Toloyl und Nitrophenyl. Bevorzugt werden Phenyl, Fluorenyl, Bromphenyl, Chlorphenyl, Toloyl und Nitrophenyl.

Beispiele sind die entsprechenden Alkoxide, lösliche Salze, und Silizium-, bzw. Titanorganische Verbindungen.

Obwohl alle Salze wie Halogenide, Nitrate und Hydroxide verwendet werden können, werden die Alkoxide, z.B. n-Butoxid, tert.- Butoxid, Isopropoxid, n-Propoxid, Ethoxid, Methoxid dieser Elemente bevorzugt.

Bevorzugt werden Titanderivate wie Tetralkoxytitanate, mit Alkylgruppen von C₁-C₁₅ wie iso-Butyl, tert-Butyl, n-Butyl, i-Propyl, Propyl, Ethyl, usw., oder andere organische Titanspezies wie Titanylacetylacetonat, Dicyclopentadienyltitandihalogenid, Titandihalogendialkoxid, Titanhalogentrialkoxid verwendet. Bei Halogensubstituenten wird Chlor bevorzugt. Es können auch Mischalkoxide von Titan mit anderen Elementen wie z. B. Titantriisopropoxd-tri-n-butylstannoxid eingesetzt werden. Die Titanvorläuferverbindungen können auch in Gegenwart von komplexbildenden Komponenten wie z. B. Acetylaceton oder Ethylacetoacetat eingesetzt werden.

Die organisch-anorganischen Silizium- und Titanvorläuferverbindungen können auch in Kombination mit anorganischen Netzwerkbildnern wie Tetraethoxysilan (Si(OC₂H₅)₄), Tetramethoxysilan (Si(OCH₃)₄) oder Homologe davon eingesetzt werden. Anstelle monomerer Alkoxide können auch deren Kondensationsprodukte verwendet werden. Kommerziell erhältlich sind z. B. Si(OC₂H₅)₄-Kondensate. Desweiteren können auch oligomere bzw. polymere Systeme wie Poly(diethoxysiloxan) eingesetzt werden.

Die genannten Silizium- und Titanverbindungen werden in Kombination mit Silanen nach der allgemeinen Formel (IIIa) oder (IIIb) eingesetzt,

[RₓSiH_{y}(OR')_{4-(x+y)}] (IIIa)

[RₓSiH_{y}(Hal)_{4-(x+y)}] (IIIb),

wobei
R und R'gleich oder verschieden sind und unabhängig voneinander ausgewählt werden aus der Gruppe C₁-C₁₂-Alkyl, C₆-C₁₂-Aryl mit x= 0,1,2,3 und y=1,2,3 und R' und R auch H sein können.

Die Si-H-haltigen Silane können auch insitu aus z.B. Halogensilanen in Gegenwart von Reduktionsmitteln wie z.B. Magnesiumhydrid generiert werden.

Die Verbindungen der allgemeinen Formeln (IIIa) und (IIIB) können ganz oder teilweise durch andere Siliziumvorläuferverbindungen mit Anteilen an Si-H-Einheiten ersetzt werden wie z. B. 1,1,3,3-Tetramethyldisiloxan, 1,3,5,7-Tetramethyl-cyclotetrasiloxan, Tri-n-hexylsilan, Triphenylsilan.

Beispiele für Silane sind Monoalkoxysilane (C1-C12), Dialkoxysilane (C1-C12), Trialkoxysilane (C1-C12), Dialkoxy-monohalogensilan (C1-C12), Monoalkoxydihalogensilan (C1-C12), Methylhydrocyclosiloxan, Trihalogensilan, Dihalogensilan, Monohalogensilan.

Bevorzugt sind Trialkoxysilane mit C1-C12, wie Trimethoxysilan, Triethoxysilan, Triisopropoxysilan, Tripropoxysilan und Tributoxysilan.

Bezogen auf Siliziumoxid liegt die molare Konzentration der Silanwasserstoffe im Cokondensat (dem Produkt aus dem Sol-Gel-Prozeß) bevorzugt im Bereich von 0,01-100 Mol-%, bevorzugt von 0,1-50 Mol- %.

Überraschend fanden wir, dass die organisch-anorganischen Hybridmaterialien mit Anteilen von Silanwasserstoffen besonders geeignet sind, Metalle wie Gold und Silber, mit hoher Dispersität und verbesserter Edelmetall-Trägerwechselwirkung auf der äußeren und inneren Oberfläche aufzubringen. Dabei werden nanoskalige Metallpartikel generiert. Auf rein anorganischen Silica- oder SiO₂-TiO₂-Mischoxid-oberflächen (analog WO-98/00415, WO-98/00414, WO-98/00413, EP-A1-0 827 779), d. h. ohne organische Modifizierung und vor allem ohne Silanwasserstoff-Anteile, ist es demgegenüber sehr viel unselektiver möglich, nanoskalige Metallpartikel mit einer sehr engen Partikelgrößenverteilung zu synthetisieren.

Überraschend haben wir gefunden, dass die erfindungsgemäßen Zusammensetzungen im Vergleich zu allen bisher bekannten Katalysatorsystemen zur katalytischen Oxidation von ungesättigten und gesättigten Kohlenwasserstoffen häufig eine um mehrere Größenordnung höhere katalytische Aktivität und Katalysatorstandzeit zeigen.

Die Reihenfolge der Arbeitsschritte bei der Sol-Gel-Synthese ist nicht festgelegt.

In einer bevorzugten Ausführungsform wird die organisch-anorganische Siliziumvorläuferverbindung in einem Lösungsmittel vorgelegt, unter Zugabe eines Katalysators mit einem Unterschuß Wasser, bezogen auf die theoretisch notwendige Menge, anhydrolysiert, anschließend die Ti-Verbindung zugegeben, bzw. vorher, gleichzeitig oder anschließend das Silan mit freien Silanwasserstoffeinheiten zugegeben und weiteres Wasser, gegebenenfalls mit Katalysator, zugesetzt. Nach der Gelbildung, die in Abhängigkeit von der Zusammensetzung, des Katalysators, der Wassermenge und der Temperatur nach wenigen Minuten bis einigen Tagen erfolgen kann, wird das Gel sofort oder nach einer Alterungszeit von bis zu 30 Tagen oder noch länger getrocknet. Gegebenenfalls können zur Vervollständigung der Hydrolyse- und Kondensationsreaktionen eine oder mehrere Behandlungen des feuchten und/oder bereits getrockneten Gels mit einem Überschuß an Wasser oder Wasserdampf erfolgen. Die Trocknung an Luft oder Inertgas erfolgt bevorzugt zwischen 50 und 250°C, besonders bevorzugt zwischen 100 und 180°C.

Die Hydrophobie der erfindungsgemäßen organisch-anorganischen Hybrid-Materialien wird maßgeblich durch die Anzahl und Art der endständigen und verbrückenden Si-C-Bindungen bestimmt. Diese haben gegenüber anderen organischen Bindungen, wie z. B. Si-O-C-Bindungen, den zusätzlichen Vorteil, dass sie weitestgehend chemisch inert sind, d. h. gegen Hydrolyse- und Oxidationsreaktionen unempfindlich sind.

Die Edelmetalle können in Form von Vorläuferverbindungen, wie Salzen oder organischen Komplexen oder Verbindungen während des Sol-Gel-Prozesses zugegeben werden, oder aber nach Herstellung des Gels, z. B. durch Fällung, Imprägnierung in Lösung, Incipient wetness, Sputtern, Kolloiden, CVD aufgebracht werden. Gegebenenfalls vorher oder anschließend an diesen Schritt schließt sich eine Oberflächenmodifizierung der Zusammensetzung an.

Die Oberflächenmodifizierung kann sowohl vor als auch nach der Edelmetallbelegung erfolgen.

Unter Modifizieren wird im Sinne der Erfindung insbesondere das Aufbringen von Gruppen ausgewählt aus Siliziumalkyl-, Siliziumaryl-, fluorhaltigen Alkyl- oder fluorhaltigen Arylgruppen auf die Oberfläche der geträgerten Zusammensetzung verstanden, wobei die Gruppen kovalent oder koordinativ an die funktionellen Gruppen (z.B. OH-Gruppen) auf der Oberfläche gebunden vorliegen. Allerdings ist auch jede andere Oberflächenbehandlung ausdrücklich im Umfang der Erfindung eingeschlossen.

Die Modifizierung kann sowohl in Flüssigphase als auch in der Gasphase erfolgen. In der Flüssigphase erfolgt die Modifizierung bevorzugt bei Temperaturen <200°C in wenig oder unpolarem Lösungsmittel, in der Gasphase wird eine Modifizierung bei Temperaturen <500°C bevorzugt. Vor der Modifizierung ist in einigen Fällen eine Vorbehandlung mit flüssigem und/oder gasförmigem Wasser vorteilhaft. Wenn die erfindungsgemäßen Zusammensetzungen mit z.B. einem Silylierungsmittel bei 200°C in der Gasphase in Kontakt gebracht werden, kann es günstig sein, die Konzentration der Silylierungsmittel durch einen Inertgasstrom zu verringem.

Die Modifizierung erfolgt bevorzugt mit siliziumorganischen und/oder fluorhaltigen siliziumorganischen bzw. organischen Verbindungen, wobei die siliziumorganischen Verbindungen bevorzugt sind.

Als siliziumorganische Verbindungen kommen alle dem Fachmann bekannten Silylierungsmittel in Frage, wie organische Silane, organische Silylamine, organische Silylamide und deren Derivate, organische Silazane, organische Siloxane und andere siliziumorganische Verbindungen, die selbstverständlich auch in Kombination eingesetzt werden können. Ebenfalls sind unter siliziumorganischen Verbindungen ausdrücklich auch Verbindungen aus Silzium und teil- oder perfluorierten organischen Resten subsummiert.

Spezielle Beispiele organischer Silane sind Chlorotrimethylsilan, Dichlorodimethylsilan, Chlorobromdimethylsilan, Nitrotrimethylsilan, Chlortrimethylsilan, Ioddimethylbutylsilan, Chlordimethylphenylsilan, Chlordimethylsilan, Dimethyl-n-propylchlorsilan, Dimethylisopropylchlorsilan, t-Butyldimethylchlorsilan, Tripropylchlorsilan, Dimethyloctylchlorsilan, Tributylchlorsilan, Trihexylchlorosilan, Dimethylethylchlorsilan, Dimethyloctadecylchlorsilan, n-Butyldimethylchlorsilan, Brommethyldimethylchlorsilan, Chlormethyldimethylchlorsilan, 3-Chlorpropyldimethylchlorsilan, Dimethoxymethylchlorsilan, Methylphenylchlorsilan, Triethoxychlorsilan, Dimethylphenylchlorsilan, Methylphenylvinylchlorsilan, Benzyldimethylchlorsilan, Diphenylchlorsilan, Diphenylmethylchlorsilan, Diphenylvinylchlorsilan, Tribenzylchlorsilan und 3-Cyanopropyldimethylchlorsilan.

Spezielle Beispiele organischer Silylamine sind N-Trimethylsilyldiethylamin, Pentafluorphenyldimethylsilylamin inklusive N-Trimethylsilylimidazole, N-t-Butyldimethylsilylimidazol, N-Dimethylethylsilylimidazol, N-Dimethyl-n-propylsilylimidazol, N-Dimethylisopropylsilylimidazol, N-Trimethylsilyldimethylamin, N-Trimethylsilylpyrrol, N-Trimethylsilylpyrrolidin, N-Trimethylsilylpiperidin und 1-Cyanoethyl(diethylamino)dimethylsilan.

Spezielle Beispiele organischer Silylamide und ihrer Derivate sind N,O-Bistrimethylsilylacetamid, N,O-Bistrimethylsilyltrifluoracetamid, N-Trimethylsilylacetamid, N-Methyl-N-trimethylsilylacetamid, N-Methyl-N-trimethylsilyltrifluoracetamid, N-Methyl-N-trimethylsilylheptafluorbutyramid, N-(t-butyldimethylsilyl)-N-trifluoracetamid und N,O-bis(diethylhydrosilyl)trifluoracetamid.

Spezielle Beispiele organischer Silazane sind Hexamethyldisilazan, Heptamethyldisilazan, 1,1,3,3-Tetramethyldisilazan, 1,3-bis(Chlormethyl)tetramethyldisilazan, 1,3-Divinyl-1,1,3,3-tetramethyldisilazan und 1,3-Diphenyltetramethyldisilazan.

Beispiele anderer siliziumorganischer Verbindungen sind N-Methoxy-N,O-Bistrimethylsilyltrifluoracetamid, N-Methoxy-N,O-bistrimethylsilycarbamat, N,O-Bistrimethylsilylsulfamat, Trimethylsilyltrifluormethansulfonat und N,N'-Bistrimethylsilylurea.

Bevorzugte Silylierungsreagenzen sind Hexamethyldisilazan, Hexamethyldisiloxan, N-Methyl-N-(Trimethylsilyl)-2,2,2-trifluoracetamid (MSTFA) und Trimethylchlorsilan.

Die erfindungsgemäßen Zusammensetzungen können bei Temperaturen von >20°C, bevorzugt bei 80 bis 240°C , besonders bevorzugt bei 120 bis 215°C eingesetzt werden. Vorteilhaft wird die Gasphasenreaktion bei erhöhten Reaktionsdrücken durchgeführt. Bevorzugt werden Reaktionsdrücke >1 bar, besonders bevorzugt 2 bis 50 bar. Die Katalysatorbelastung beträgt zwischen 0,5 und 40 Liter Gas pro Gramm Katalysator und Stunde.

Überraschend haben wir gefunden, dass die erfindungsgemäßen Zusammensetzungen enthaltend Gold- und/oder Silberpartikel und titanhaltige, organisch-anorganische Hybridmaterialien, dadurch gekennzeichnet, dass die Zusammensetzungen Si-H-Gruppen enthalten, im Vergleich zu bisher bekannten Katalysatorsystemen zur katalytischen Oxidation von Alkenen und Alkanen eine um mehrere Größenordnung höhere katalytische Aktivität und hohe Katalysatorstandzeiten zeigen.

Daher stellt die Verwendung der erfindungsgemäßen geträgerten Zusammensetzungen zur Oxidation von Kohlenwasserstoffen eine weitere Lösung der gestellten Aufgabe und auch einen weiteren Gegenstand der Erfindung dar.

Die nach Monaten geringfügig desaktivierten Katalysatoren lassen sich häufig sowohl thermisch als auch durch Waschen mit geeigneten Lösungsmitteln, wie z.B. Alkohole, oder mit verdünnten Wasserstoffperoxidlösungen (z. B. 3-10 %ige H₂O₂-Methanol-Lösung) wieder regenerieren.

Die erfindungsgemäße Zusammensetzung kann grundsätzlich auf alle Kohlenwasserstoffe angewendet werden. Unter dem Begriff Kohlenwasserstoff werden ungesättigte oder gesättigte Kohlenwasserstoffe wie Olefine oder Alkane verstanden, die auch Heteroatome wie N, O, P, S oder Halogene enthalten können. Die zu oxidierende organische Komponente kann azyklisch, monozyklisch, bizyklisch oder polyzyklisch und kann monoolefinisch, diolefinisch oder polyolefinisch sein. Bei organischen Komponenten mit zwei oder mehreren Doppelbindungen können die Doppelbindungen konjugiert und nichtkonjugiert vorliegen. Bevorzugt werden Kohlenwasserstoffe oxidiert, aus denen solche Oxidationsprodukte gebildet werden, deren Partialdruck niedrig genug liegt, um das Produkt ständig vom Katalysator zu entfernen. Bevorzugt sind ungesättigte und gesättigte Kohlenwasserstoffe mit 2 bis 20, vorzugsweise 2 bis 10 Kohlenstoffatomen, insbesondere Ethen, Ethan, Propen, Propan, Isobutan, Isobutylen, 1-Buten, 2-Buten, cis-2-Buten, trans-2-Buten, 1,3-Butadien, Penten, Pentan, 1-Hexen, Hexan, Hexadien, Cyclohexen, Benzol.

Die geträgerten Zusammensetzungen können hierbei in jeder beliebigen physikalischen Form für Oxidationsreaktionen eingesetzt werden, z.B. gemahlene Pulver, spherische Partikel, Pellets, Extrudate, Granulate.

Eine bevorzugte Verwendung ist die Gasphasenreaktion von Sauerstoff mit Wasserstoff in Gegenwart der erfindungsgemäßen geträgerten Zusammensetzungen. Hierbei werden selektiv aus Olefinen Epoxide, aus gesättigten sekundären Kohlenwasserstoffen Ketone und aus gesättigten tertiären Kohlenwasserstoffen Alkohole erhalten. Die Katalysatorstandzeiten liegen je nach verwendetem Edukt bei einigen Wochen, Monaten, oder länger.

Das relative molare Verhältnis von Kohlenwasserstoff, Sauerstoff, Wasserstoff und optional einem Verdünnungsgas ist in weiten Bereichen variierbar.

Die molare Menge des eingesetzten Kohlenwasserstoffs in Bezug auf die Gesamtmolzahl aus Kohlenwasserstoff, Sauerstoff, Wasserstoff und Verdünnungsgas kann in weiten Bereichen variiert werden. Bevorzugt wird ein Überschuß von Kohlenwasserstoff, bezogen auf eingesetzten Sauerstoff (auf molarer Basis) eingesetzt. Der Kohlenwasserstoffgehalt liegt typischerweise größer 1 Mol% und kleiner als 95 Mol%. Bevorzugt werden Kohlenwasserstoffgehalte im Bereich von 5 bis 85 Mol-%, besonders bevorzugt von 10 bis 80 Mol-% eingesetzt.

Der Sauerstoff kann in verschiedenster Form eingesetzt werden, z.B. molekularer Sauerstoff, Luft und Stickstoffoxid. Molekularer Sauerstoff wird bevorzugt. Der molare Sauerstoffanteil - in Bezug auf die Gesamtmolzahl aus Kohlenwasserstoff, Sauerstoff, Wasserstoff und Verdünnungsgas - kann in weiten Bereichen variiert werden. Bevorzugt wird der Sauerstoff im molaren Unterschuß zum Kohlenwasserstoff eingesetzt. Bevorzugt werden im Bereich von 1-30 Mal%, besonders bevorzugt 5-25 mol% Sauerstoff eingesetzt.

In Abwesenheit von Wasserstoff zeigen die erfindungsgemäßen geträgerten Zusammensetzungen nur sehr geringe Aktivität und Selektivität. Bis 180°C ist die Produktivität in Abwesenheit von Wasserstoff gering, bei Temperaturen größer 230°C werden neben Partialoxidationsprodukten größere Mengen Kohlendioxid gebildet. Es kann jede bekannte Wasserstoffquelle genutzt werden, wie z.B. reiner Wasserstoff, Cracker-Wasserstoff, Synthesegas oder Wasserstoff aus Dehydrierung von Kohlenwasserstoffen und Alkoholen. In einer anderen Ausführungsform der Erfindung kann der Wasserstoff auch in einem vorgeschalteten Reaktor in situ erzeugt werden, z.B. durch Dehydrierung von Propan oder Isobutan oder Alkoholen wie z.B. Isobutanol. Der Wasserstoff kann auch als Komplex-gebundene Spezies, z.B. Katalysator-Wasserstoffkomplex, in das Reaktionssystem eingeführt werden. Der molare Wasserstoffanteil - in Bezug auf die Gesamtmolzahl aus Kohlenwasserstoff, Sauerstoff, Wasserstoff und Verdünnungsgas - kann in weiten Bereichen variiert werden. Typische Wasserstoffgehalte liegen bei größer als 0,1 Mol-%, bevorzugt bei 2-80 Mol-%, besonders bevorzugt bei 5-80 Mol-%.

Zu den essentiell notwendigen oben beschriebenen Eduktgasen kann optional auch ein Verdünnungsgas, wie Stickstoff, Helium, Argon, Methan, Kohlendioxid, Kohlenmonoxid oder ähnliche, sich überwiegend inert verhaltende Gase, eingesetzt werden. Auch Mischungen der beschriebenen Inertkomponenten können eingesetzt werden. Der Inertkomponentenzusatz ist zum Transport der freiwerdenden Wärme dieser exothermen Oxidationsreaktion und aus sicherheitstechnischen Gesichtspunkten günstig. Wird der erfindungsgemäße Prozeß in der Gasphase durchgeführt, werden bevorzugt gasförmige Verdünnungskomponenten wie z.B. Stickstoff, Helium, Argon, Methan und evtl. Wasserdampf und Kohlendioxid verwendet. Wasserdampf und Kohlendioxid sind zwar nicht völlig inert, bewirken aber bei sehr kleinen Konzentrationen (<2 Vol.-%) einen positiven Effekt.

Bei Ausführung der Erfindung in der Flüssigphase wird zweckmäßigerweise eine oxidationsstabile und thermisch stabile inerte Flüssigkeit gewählt (z.B. Alkohole, Polyalkohole, Polyether, halogenierte Kohlenwasserstoffe, Silikonöle). Die erfindungsgemäßen geträgerten Zusammensetzungen sind auch in der Flüssigphase zur Oxidation von Kohlenwasserstoffe geeignet. Sowohl in Gegenwart von organischen Hydroperoxiden (R-OOH) werden z. B. Olefine in der Flüssigphase hochselektiv an den beschriebenen Katalysatoren zu Epoxiden umgesetzt, als auch in Gegenwart von Wasserstoffperoxid oder in Gegenwart von Sauerstoff und Wasserstoff werden Olefine in der Flüssigphase hochselektiv an den beschriebenen Katalysatoren zu Epoxiden umgesetzt.

Wir haben gefunden, dass die oben beschriebenen selektive Oxidationsreaktion eine große Katalysator-Struktursensitivität aufweist. Bei Vorliegen von nanodispersen Gold- und/oder Silberpartikeln in der geträgerten Zusammensetzung wurde eine vorteilhafte Erhöhung der Produktivität zum selektiven Oxidationsprodukt beobachtet.

Die Eigenschaften des Trägers lassen sich außerdem durch den Einbau von anderen oxophilen Elementen als Silicium wie Bor, Aluminium, Yttrium, Tantal, Zirkon oder Titan vorteilhaft beeinflussen. Die Auswahl dieser Heteroatome ist erfindungsgemäß auf Elemente beschränkt, die über redoxstabile Oxidationsstufen verfügen.

Die Polarität der erfindungsgemäßen Katalysatoren lässt sich neben dem atomaren Einbau von Kohlenstoff zusätzlich durch den erfindungsgemässen Einbau von Si-H-Einheiten sensibel einstellen. Die erfindungsgemässen Si-H- und eventuell vorhandene Si-C-Gruppen stellen ein ideales Werkzeug zur Einstellung der äußeren und inneren Polarität für die partielle Oxidation dar.

Das räumlich enge Zusammenspiel von Gold und/oder Silber und Ti-Zentren auf dem organisch-anorganischen Träger mit Si-H Gruppen arbeitet besonders effizient, d.h. es werden exzellente Epoxidationskatalysatoren in Gegenwart von Sauerstoff und Wasserstoff erhalten.
Die erfindungsgemäßen Zusammensetzungen lassen sich verfahrenstechnisch problemlos und kostengünstig im technischen Maßstab herstellen.

Die charakteristischen Eigenschaften der vorliegenden Erfindung werden an Hand von Katalysatorpräparationen und katalytischen Testreaktion in den folgenden Beispielen veranschaulicht.

Es versteht sich von selbst, dass die Erfindung nicht auf die nachfolgenden Beispiele beschränkt ist.

### Beispiele

### Vorschrift zum Test der Katalysatoren (Testvorschrift)

Es wurde ein Metallrohrreaktor mit 10 mm Innendurchmesser und 20 cm Länge eingesetzt, welcher mittels eines Ölthermostaten temperiert wurde. Der Reaktor wurde mit einem Satz von vier Massendurchflußregler (Kohlenwasserstoff, Sauerstoff, Wasserstoff, Stickstoff) mit Eduktgasen versorgt. Zur Reaktion wurden 500 mg Katalysator bei 140°C und Normaldruck vorgelegt. Die Eduktgase wurden von oben in den Reaktor eindosiert. Die Standardkatalysatorbelastung lag bei 3 l Gas / (g Kat. * h). Als "Standardkohlenwasserstoff" wurde Propen beispielhaft ausgewählt. Zur Durchführung der Oxidationsreaktionen wurde ein mit Stickstoff angereicherter Gasstrom, nachfolgend immer als Standard-Gaszusammensetzung bezeichnet, ausgewählt: N₂/H₂/O₂/C₃H₆ : 14 / 75 / 5 / 6 %. Die Reaktionsgase wurden gaschromatographisch quantitativ analysiert. Die gaschromatographische Auftrennung der einzelnen Reaktionsprodukte erfolgte durch eine kombinierte FID/WLD-Methode, bei der drei Kapillarsäulen durchlaufen werden:
FID: HP-Innowax, 0,32 mm Innendurchmesser, 60 m lang, 0,25 µm Schichtdicke.
WLD: Hintereinanderschaltung von
HP-Plot Q, 0,32 mm Innendurchmesser, 30 m lang, 20 µm Schichtdicke
HP-Plot Molsieve 5 A, 0,32 mm Innendurchmesser, 30 m lang, 12 µm Schichtdicke.

### Beispiel 1

Dieses Beispiel beschreibt die Präparation eines Katalysators, bestehend aus einem Silizium- und titanhaltigen, organisch-anorganischen Hybridmaterial mit freien Silanwasserstoffeinheiten, welches mit Goldteilchen (0,1 Gew.-%) über Incipient-wetness belegt wurde. Bezogen auf Silizium beträgt der Gehalt an nicht-hydrolysierbaren organischen Komponenten 68 Mol-%, der an Silanwasserstöffen 31% und der von Titan 4 Mol-%.

10,1 g Methyltrimethoxysilan (74,1 mmol) und 15 g Ethanol (p.A.) wurden mit 1,9 g einer 0,1 n Lösung von p-Toluolsulfonsäure in Wasser versetzt und die Mischung 2 Stunden gerührt. Anschließend wurden 1,46 g Tetrabutoxytitan (4,3 mmol) langsam zugegeben, die Mischung weitere 30 Minuten gerührt, eine Lösung von 5,6 g Triethoxysilan (34,1 mmol) zugegeben, erneut 30 Minuten gerührt, unter Rühren mit einer Mischung von 1,23 g einer 0,1 n Lösung von p-Toluolsulfonsäure in Wasser versetzt und schließlich stehengelassen. Der Ansatz erreicht nach ca. 7 min den Gelpunkt. Nach einer Alterungszeit von 24 h wurde das Gel gemörsert und 8 Stunden bei 120°C unter Luft getrocknet.

5,4 g Sol-Gel-Material wurde mit einer Lösung, bestehend aus 540 mg einer 1 %igen methanolischen Goldlösung (HAuCl₄ x 3 H₂O; Firma Merck), welche mit Methanol auf 2,8 g aufgefüllt wurde, imprägniert, das makroskopisch trockene Material 4 h bei Raumtemperatur getrocknet und anschließend 2 h bei 400°C unter Stickstoffstrom getempert.

In einem Test gemäß der Testvorschrift wurde eine konstante PO-Selektivitäten von 95 % erreicht. Die PO-Maximalausbeute von 8 %, welche nach 8 h erreicht wurde, pendelte sich nach 10 Tagen auf 7,4 % ein.

### Beispiel 2

Dieses Beispiel beschreibt die Präparation eines Katalysators analog Beispiel 1, aber das Trägermaterial wird vor der Goldbelegung oberflächenmodifiziert.

Zur Oberflächenmodifizierung wurden unter Schutzgas 5 g getrocknetes Sol-Gel-Material mit 5 g 1,1,1,3,3,3-Hexamethyldisilazan in 50 g trockenem n-Hexan vorgelegt und unter Rühren für 2 Stunden unter Rückfluß erhitzt. Anschließend wurde die überstehende Lösung abdekantiert, der Rückstand 2 mal mit je 100 ml n-Hexan gewaschen, unter Vakuum von flüchtigen Bestandteile befreit und 4 Stunden bei 150°C getrocknet.

In einem Test gemäß der Testvorschrift wurde eine konstante PO-Selektivitäten von 95 % erreicht. Die PO-Maximalausbeute von 7,5 %, welche nach 13 h erreicht wurde, pendelte sich nach 10 Tagen auf 7,2 % ein.

### Beispiel 3

Dieses Beispiel beschreibt die Präparation eines Katalysators analog Beispiel 1, aber das bei Raumtemperatur getrocknete edelmetallhaltige Material wird 2 h bei 400°C unter Wasserstoffatmosphäre getempert.

In einem Test gemäß der Testvorschrift wurde eine konstante PO-Selektivitäten von 95 % erreicht. Die PO-Maximalausbeute von 8,2 %, welche nach 10 h erreicht wurde, pendelte sich nach 10 Tagen auf 7,6 % ein.

### Beispiel 4

Dieses Beispiel beschreibt die Präparation eines Katalysators bestehend aus einem Silizium- und titanhaltigen, organisch-anorganischen Hybridmaterial mit freien Silanwasserstoffeinheiten, welches mit Goldteilchen (0,1 Gew.-%) über Incipient-wetness belegt wurde. Bezogen auf Silizium beträgt der Gehalt an nicht-hydrolysierbaren organischen Komponenten 94 Mol-%, der an Silanwasserstoffen 5,5 % und der von Titan 3,9 Mol-%.

10,1 g Methyltrimethoxysilan (74,1 mmol) und 15 g Ethanol (p.A.) wurden mit 1,9 g einer 0,1 n Lösung von p-Toluolsulfonsäure in Wasser versetzt und die Mischung 2 Stunde gerührt. Anschließend wurden 0,9 g Tetrapropoxytitan (3,1 mmol) langsam zugegeben, die Mischung weitere 30 Minuten gerührt, eine Lösung von 0,7 g Triethoxysilan (4,3 mmol) zugegeben, erneut 30 Minuten gerührt, unter Rühren mit einer Mischung von 0,34 g einer 0,1 n Lösung von p-Toluolsulfonsäure in Wasser versetzt und schließlich stehengelassen. Der Ansatz erreicht nach ca. 20 h den Gelpunkt. Nach einer Alterungszeit von 60 h wurde das Gel gemörsert und 5 Stunden bei 120°C unter Luft getrocknet.

2,7 g Sol-Gel-Material wurde mit einer Lösung, bestehend aus 270 mg einer 1%igen methanolischen Goldlösung (HAuCl₄ x 3 H₂O; Firma Merck), welche mit Methanol auf 1,4 g aufgefüllt wurde, imprägniert, das Material 4 h bei Raumtemperatur getrocknet und anschließend 2 h bei 400°C unter Stickstoffatmosphäre getempert.

In einem Test gemäß der Testvorschrift wurde eine konstante PO-Selektivitäten von 95 % erreicht. Die PO-Maximalausbeute von 7 %, welche nach 12 h erreicht wurde, pendelte sich nach 10 Tagen auf 6,8 % ein.

### Beispiel 5

Dieses Beispiel beschreibt die Präparation eines Katalysators, bestehend aus einem Silizium- und titanhaltigen, organisch-anorganischen Hybridmaterial mit freien Silanwasserstoffeinheiten, welches mit Goldteilchen (0,5 Gew.-%) nach der Abscheidung-Ausfällungsmethode (deposition-precipitation) belegt wurde. Bezogen auf Silizium beträgt der Gehalt an nicht-hydrolysierbaren organischen Komponenten 68 Mol-%, der an Silanwasserstoffen 8,7 % und der von Titan 4 Mol-%.

Der Sol-Gel-Ansatz erfolgt analog Beispiel 1.

2 g Träger wurden in 15 ml Methanol (Merck, p. a.) vorgelegt, mit 20 mg HAuCl₄ x 3 H₂O (0,1 mmol, Firma Merck), gelöst in 5 ml Methanol, versetzt, mit 0.5 ml 1 n Na₂CO₃-Lösung auf pH 8 eingestellt, 30 min gerührt, 2 ml Mononatriumcitratlösung (32,1 g/l; pH = 8) zugegeben, erneut pH-Wert kontrolliert, 60 min gerührt, Feststoffabtrennung, 3 x mit je 20 ml Methanol gewaschen, 10 h bei 120°C bei Normaldruck getrocknet, 5 h bei 200°C unter Luft calciniert und anschließend 2 h bei 400 °C unter Stickstoff getempert. Der Goldgehalt des Gold-Titan-Siliciumkatalysators beträgt 0,48 Gew.-% (ICP-Analyse).

In einem Test gemäß der Testvorschrift wurde eine konstante PO-Selektivitäten von 95 % erreicht. Die PO-Maximalausbeute von 4,5 %, welche nach 5 h erreicht wurde, pendelte sich nach 10 Tagen auf 2,9 % ein.

### Beispiel 6

Dieses Beispiel beschreibt die Präparation eines Katalysators analog Beispiel 1, aber vor der Edelmetallbelegung wurde der Katalysatorträger gemahlen.

Zur Mahlung wurde das titanhaltige getrocknete Material in Wasser gegeben, in einer Perlmühle (Al₂O₃-Perlen von 0,2-0,4 mm Durchmesser) 1 h bei 1500 Umin⁻¹ gemahlen, das Lösungsmittel entfernt, das Pulver 4 Stunden bei 120°C bei Normal druck getrocknet, und anschließend mit 0,1 Gew.% Gold analog Beispiel 1 belegt.

In einem Test gemäß der Testvorschrift wurde eine konstante PO-Selektivitäten von 95 % erreicht. Die PO-Maximalausbeute von 8,7 %, welche nach 5 h erreicht wurde, pendelte sich nach 10 Tagen auf 7,9 % ein.

### Beispiel 7

Dieses Beispiel beschreibt die Präparation eines Katalysators analog Beispiel 1, aber der Test gemäß der Testvorschrift erfolgt bei 4 bar abs.

In einem Test gemäß der Testvorschrift bei 4 bar abs. wurde eine konstante PO-Selektivitäten von 95 % erreicht. Die PO-Maximalausbeute von 10,4 %, welche nach 7 h erreicht wurde, pendelte sich nach 10 Tagen auf 9,8 % ein.

### Beispiel 8

Dieses Beispiel beschreibt die Präparation eines Katalysators analog Beispiel 1, aber 60 min nach der Zugabe von Tetrabutoxytitan wird der homogene Ansatz mit 0,7 g Ta(OEt)₅ (1,5 mmol, Firma Chempur, 99,9 %ig) versetzt, 15 min gerührt und analog Beispiel 1 mit Triethoxysilan versetzt, geliert, aufgearbeitet, mit Gold belegt und getempert.

In einem Test gemäß der Testvorschrift wurde eine konstante PO-Selektivitäten von 95 % erreicht. Die PO-Maximalausbeute von 8,1 %, welche nach 4 h erreicht wurde, pendelte sich nach 10 Tagen auf 7,6 % ein.

### Beispiel 9

Dieses Beispiel beschreibt die Präparation eines Katalysators analog Beispiel 1, aber es wurde ein löslicher Gold-Cyanid-Precursor eingesetzt.

5,4 g Sol-Gel-Material wurde mit einer Lösung, bestehend aus 540 mg einer 1%igen methanolischen Goldlösung (HAuCl₄ x 3 H₂O; Firma Merck) und 2 mg Natriumcyanid welche mit Methanol auf 2,8 g aufgefüllt wurde, imprägniert, das Material 4 h bei Raumtemperatur getrocknet und anschließend 2 h bei 400°C unter Stickstoffatmosphäre getempert.

In einem Test gemäß der Testvorschrift wurde eine konstante PO-Selektivitäten von 97 % erreicht. Die PO-Maximalausbeute von 5 %, welche nach 8 h erreicht wurde, pendelte sich nach 10 Tagen auf 4,9 % ein.

### Beispiel 10

Dieses Beispiel beschreibt die Präparation eines Katalysators analog Beispiel 1, aber die Goldvorläuferlösung wurde mit Polyvinylpyrolidon versetzt.

5,4 g Sol-Gel-Material wurde mit einer Lösung, bestehend aus 540 mg einer 1%igen methanolischen Goldlösung (HAuCl₄ x 3 H₂O; Firma Merck) und 3 mg Polyvinylpyrolidon welche mit Methanol auf 2,8 g aufgefüllt wurde, imprägniert, das Material 4 h bei Raumtemperatur getrocknet und anschließend 2 h bei 400°C unter Stickstoffatmosphäre getempert.

In einem Test gemäß der Testvorschrift wurde eine konstante PO-Selektivitäten von 94 % erreicht. Die PO-Maximalausbeute von 3,5 %, welche nach 3 h erreicht wurde, pendelte sich nach 10 Tagen auf 2,5 % ein.

### Beispiel 11

Dieses Beispiel beschreibt die Präparation eines Katalysators, bestehend aus einem Silizium- und titanhaltigen, organisch-anorganischen Hybridmaterial mit freien Silanwasserstoffeinheiten, welches mit Silberteilchen (1 Gew.-%) über Incipient-wetness belegt wurde. Bezogen auf Silizium beträgt der Gehalt an nicht-hydrolysierbaren organischen Komponenten 68 Mol-%, der an Silanwasserstoffen 31,5 % und der von Titan 4 Mol-%. Die Katalysatorherstellung erfolgte analog Beispiel 1. Anstatt mit Goldteilchen wird der Katalysatorträger mit Silberteilchen belegt.

5,4 g Sol-Gel-Material wurde mit einer Lösung, bestehend aus 540 mg einer 10%igen wäßrigen Silberlösung (AgNO₃; Firma Merck), welche mit Methanol auf 2,8 g aufgefüllt wurde, imprägniert, das Material 4 h bei Raumtemperatur getrocknet und anschließend 2 h bei 400°C unter Stickstoffatmosphäre getempert.

In einem Test gemäß der Testvorschrift wurde eine konstante PO-Selektivitäten von 92 % erreicht. Die PO-Maximalausbeute von 0,5 %, welche nach 2 h erreicht wurde, pendelte sich nach 50 h auf 0,4 % ein.

### Beispiel 12

Dieses Beispiel beschreibt die Präparation eines Katalysators analog Beispiel 11, aber die Temperung des silberhaltigen Material erfolgt bei 400 °C unter Wasserstoffatmosphäre.

In einem Test gemäß der Testvorschrift wurde eine konstante PO-Selektivitäten von 92 % erreicht. Die PO-Maximalausbeute von 0,8 %, welche nach 2 h erreicht wurde, pendelt sich nach 50 h auf 0,6 % ein.

### Beispiel 13

Trans-2-buten wird anstelle von Propen als ungesättigter Kohlenwasserstoff eingesetzt. Zur partiellen Oxidation von Trans-2-buten wird ein Katalysator analog Beispiel 1 verwendet.

In einem Test gemäß der Testvorschrift wurde eine konstante Epoxid-Selektivität von 94 % erreicht. Die Butylenoxid-Maximalausbeute von 7 %, welche nach 6 h erreicht wurde, pendelte sich nach 10 Tagen auf 6,5 % ein.

### Beispiel 14

Cyclohexen wird anstelle von Propen als ungesättigter Kohlenwasserstoff ausgewählt. Zur partiellen Oxidation von Cyclohexen wird ein Katalysator analog Beispiel 1 verwendet. Cyclohexen wird mit Hilfe eines Verdampfers in die Gasphase gebracht.

In einem Test gemäß der Testvorschrift wurde eine konstante Epoxid-Selektivität von 94 % erreicht. Die Hexenoxid-Maximalausbeute von 6,5 %, welche nach 7 h erreicht wurde, pendelte sich nach 10 Tagen auf 6,3 % ein.

### Beispiel 15

1,3-Butadien wird anstelle von Propen als ungesättigter Kohlenwasserstoff ausgewählt. Zur partiellen Oxidation von 1,3-Butadien wird ein Katalysator analog Beispiel 1 verwendet.

In einem Test gemäß der Testvorschrift wurde eine Butenmonooxid-Selektivität von 85 % erreicht. Die Butenmonooxid-Maximalausbeute von 2,8 %, welche nach 5 h erreicht wurde, pendelte sich nach 10 Tagen auf 2,3 % ein.

### Beispiel 16

Propan wird anstelle von Propen als gesättigter Kohlenwasserstoff eingesetzt. Zur partiellen Oxidation von Propan wird ein Katalysator analog Beispiel 1 verwendet.

In einem Test gemäß der Testvorschrift wurde eine Aceton-Selektivität von 80 % erreicht. Die Aceton-Maximalausbeute von 1,5 %, welche nach 3 h erreicht wurde, pendelt sich nach 50 h auf 1,0 % ein.

### Beispiel 17: Vergleichendes Beispiel gemäß EP-A1-827771

Dieses Beispiel beschreibt die Präparation eines hydrophilen, rein anorganischen Katalysatorträgers analog EP-A1-827771, bestehend aus den Oxiden von Silicium und Titan, welcher mit Goldteilchen durch deposition-precipitation belegt wird. Der Ti-haltige anorganische Katalysatorträger wird durch Imprägnierung von pyrogenem, rein anorganischen Silica mit Titanylacetylacetonat erhalten.

30 g Aerosil 200 (pyrogenes Siliciumdioxid, Degussa, 200 m²/g) werden in 250 ml trockenem Methanol suspendiert, mit 0,98 g Titanylacetylacetonat (3,9 mmol, Firma Merck) versetzt und 2 h bei Raumtemperatur gerührt. Am Rotationsverdampfer wird die Suspension zur Trockene eingeengt, der Feststoff wird anschließend bei 130°C getrocknet und bei 600°C im Luftstrom für 3 h calciniert.

0,16 g Tetrachlorogoldsäure (0,4 mmol, Firma Merck) wird in 500 ml destilliertem Wasser gelöst, mit einer 2 n Natriumhydroxidlösung auf pH 8,8 eingestellt, auf 70°C erwärmt, mit 10 g von obigem titanhaltigen Silica versetzt und 1 h gerührt. Der Feststoff wird abfiltriert, mit 30 ml destilliertem Wasser gewaschen, bei 120°C 10 h getrocknet und 3 h bei 400°C an der Luft calciniert. Nach ICP-Analyse weist der Katalysator 0,45 Gew.-% Gold auf.

In einem Test gemäß der Testvorschrift wurden bei PO-Selektivitäten von 92 % nach 20 min ein Propenumsatz von 2,3 Mol-%, nach 100 min ein Propenumsatz von 1,5 Mol-%, nach 4 h ein Propenumsatz von 1,0 Mol-% und nach 50 h ein Propenumsatz von 0,5 Mol-% erreicht. Die Katalysatordesaktivierung nahm mit zunehmender Zeit weiter zu.

### Beispiel 18: Vergleichendes Beispiel gemäß WO 98/00413

Dieses Beispiel beschreibt die Präparation eines rein anorganischen kristallinen Titansilikalitkatalysatorträgers (TS 1), bestehend aus den Gerüstoxiden von Silizium und Titan, welcher analog WO 98/00413 mit Gold belegt wurde. Der TS 1-Katalysatorträger von der Firma Leuna wurde durch Hydrothermalsynthese erhalten. Das anorganische Si- und Ti-Gerüstsilikat weist eine MFI-Struktur auf (XRD) und mittels Raman.Spektroskopie konnte gezeigt werden, dass das Material keine kristallinen Titandioxidphasen enthält.

10,04 g TS 1 (Firma Leuna) werden analog WO 98/00413 in eine wäßrige Tetrachlorogoldsäurelösung (0,483 g HAuCl₄*3 H₂O in 50 ml Wasser) suspendiert, der pH-Wert mit 2 n Na₂CO₃-Lösung auf pH 7,8 eingestellt, 1,97 g Magnesiumnitrat (Mg(NO₃)₂*6H₂O) zugegeben, der pH-Wert erneut mit 2n Na₂CO₃₋-Lösung auf pH 7,8 eingestellt, 8 h gerührt, der Feststoff abfiltriert, 3 x mit je 150 ml H₂O gewaschen, 2 h bei 100°C getrocknet, innerhalb von 8 h auf 400°C aufgeheizt und 5 h bei 400°C gehalten. Der rein anorganische Katalysator enthält 0.95 Gew.% Gold (ICP).

In einem Test gemäß der Testvorschrift wurden bei PO-Selektivitäten von 92 % nach 20 min ein Propenumsatz von 2,5 %, nach 100 min ein Propenumsatz von 1,7 %, nach 4 h ein Propenumsatz von 1,6%, nach 10 h ein Propenumsatz von 1,5 % und nach 100 h ein Propenumsatz von 1,1 % erreicht. Die Katalysatordesaktivierung nahm mit zunehmender Zeit weiter zu.

### Untersuchungen der Katalysatoren

Die Katalysatoren können vorteilhaft durch sogenannte DRIFTS-Spektroskopie charakterisiert werden. DRIFTS (Diffuse Reflectance Infrared Fourier Transform Spectroscopy) ist eine gut etablierte schwingungsspektroskopische Methode zur strukturellen Charakterisierung von funktionellen Gruppen und Adsorbaten an Festkörperoberflächen. Angaben zum Prinzip der Methode und einige Anwendungsbeispiele aus dem Gebiet der heterogenen Katalyse finden sich z.B. im Artikel von Mestl, G., Knözinger, H., im Handbook of Heterogeneous Catalysis, Vol. 2, S. 539 ff. (VCH, Weinheim 1997), und der darin zitierten Literatur.

Zur Charakterisierung der erfindungsgemäßen Katalysatormaterialien wurden entsprechende Proben für einige Stunden bei 200°C im Trockenschrank aufbewahrt, im heißen Zustand in eine Inertgaszelle überführt und ohne weiteren Luftkontakt (zur Vermeidung von H₂O-Readsorption an der Probenoberfläche) mittels DRIFTS spektroskopisch untersucht.

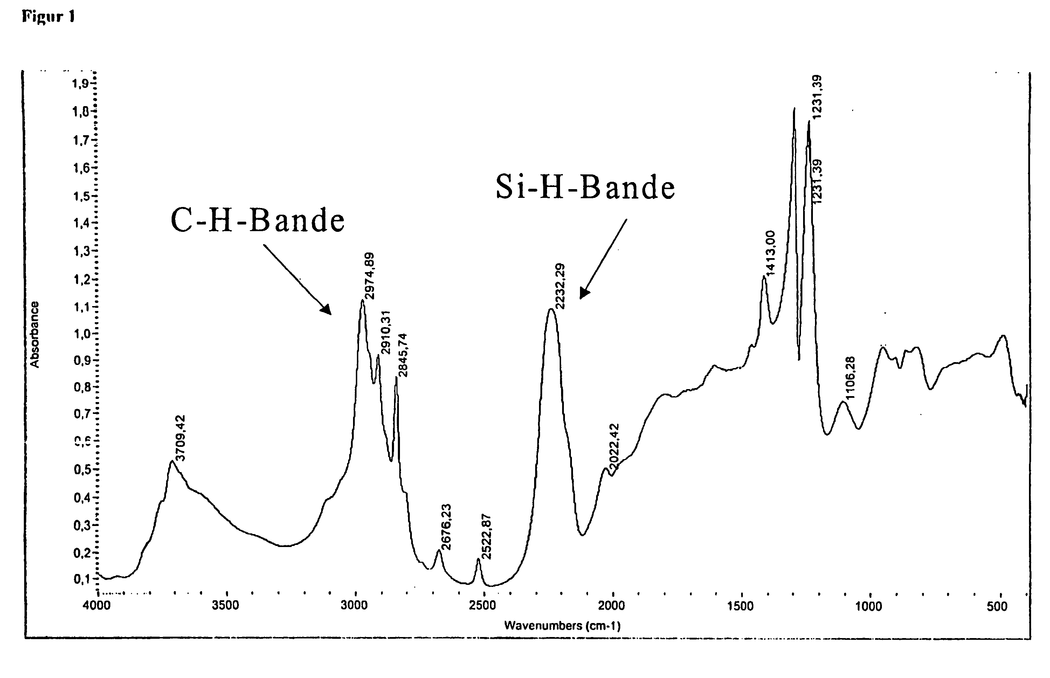

Figur 1 zeigt das DRIFT-Spektrum einer Zusammensetzung enthaltend Goldpartikel auf einem titanhaltigen, organisch-anorganischen Hybridmaterial, dadurch gekennzeichnet, dass die Zusammensetzung Anteile von Silanwasserstoffen enthält. Das Beispiel zeigt eine Zusammensetzung, die nicht nachträglich oberflächenmodifiziert wurde (z. B. mit Silylierungsmitteln).

Die deutlich hervortretenden Banden um 3000 cm⁻¹ im Spektrum der Zusammensetzung sind der Kohlenwasserstoffbelegung (CH₃ - Gruppen), die um 2230 cm⁻¹ den Si-H-Einheiten zuzuordnen.

## Patentansprüche

1. Verfahren zur Herstellung einer Zusammensetzung umfassend die Schritte
1) Herstellung eines organisch-anorganischen Hybridmaterials, das Siliziumoxid, 0,1 - 20 Mol.% Titan in oxidischer Form, bezogen auf das Siliziumoxid und Si-H-Gruppen auf der äußeren und inneren Oberfläche, enthält und über einen Sol-Gel-Prozess hergestellt wird bei dem Silane der allgemeinen Formel (IIIa) oder (IIIb),
[RₓSiH_{y}(OR')_{4-(x+y)}] (IIIa)
[RₓSiH_{y}(Hal)_{4-(x+y)}] (IIIb)
wobei
R und R' gleich oder verschieden sind und unabhängig voneinander ausgewählt werden aus der Gruppe C₁-C₁₂-Alkyl, C₆-C₁₂-Aryl mit x = 0, 1, 2, 3 und y = 1, 2 ,3 und R' und R auch H sein können,
eingesetzt werden, und anschließend
2) eine ein- oder mehrfach Behandlung des organisch-anorganischen Hybridmaterials mit einer löslichen Gold und/oder Silberverbindung erfährt.

2. Verfahren nach Anspruch 1, **dadurch gekennzeichnet, dass** geeignete niedermolekulare Verbindungen in einem Lösungsmittel gemischt werden und anschließend durch Zugabe von Wasser und sauren oder basischen Katalysatoren die Hydrolyse- und/oder Kondensationsreaktion eingeleitet wird.

3. Verfahren nach Anspruch 2, **dadurch gekennzeichnet, dass** als Katalysator eine oder mehrere organische Säuren eingesetzt werden.

4. Verfahren nach einem oder mehreren der Ansprüche 1 bis 3, **dadurch gekennzeichnet dass** die Zusammensetzung in einem Zwischen- oder Endschritt bei Temperaturen im Bereich von 100 - 1000°C getempert werden.

5. Verfahren nach Anspruch 4, **dadurch gekennzeichnet, dass** die Temperung bei Temperaturen im Bereich von 200 - 600°C unter Inertgas durchgeführt wird.

6. Verfahren nach Anspruch 1, **dadurch gekennzeichnet, dass** das Volumen der Lösung auf dem Träger kleiner oder gleich dem Porenvolumen des titanhaltigen, organisch-anorganischen Hybridmaterials mit Si-H-Gruppen ist.

7. Zusammensetzungen erhältlich durch das Verfahren nach Anspruch 1.

8. Zusammensetzungen nach Anspruch 7 **dadurch gekennzeichnet, dass** die organisch-anorganischen Hybridmaterialien organisch modifizierte Gläser sind und im Netzwerk terminale und/oder verbrückende organische Gruppen enthalten.

9. Zusammensetzung nach Anspruch 7 und/oder 8 **dadurch gekennzeichnet, dass** die molare Konzentration der Si-H-Gruppen bezogen auf den Siliziumoxidgehalt im Bereich von 0,01-100 Mol- % liegt.

10. Zusammensetzung nach einem oder mehreren der Ansprüche 7 bis 9, **dadurch gekennzeichnet, dass** das organisch-anorganische Hybridmaterial bezogen auf Siliziumoxid zwischen 0,1 und 10 Mol-% Titan sowie gegebenenfalls weitere Fremdoxide, sogenannte Promotoren, enthält.

11. Zusammensetzung nach einem oder mehreren der Ansprüche 7 bis 10, **dadurch gekennzeichnet, dass** sie zwischen 0,001 und 4 Gew.-% Gold oder zwischen 0,01 und 20 Gew.-% Silber oder eine Mischung aus Gold und Silber enthält.

12. Zusammensetzung nach einem oder mehreren der Ansprüche 7 bis 11, **dadurch gekennzeichnet, dass** deren Oberfläche mit Siliziumalkyl(aryl)-Verbindungen modifiziert wurde.

13. Verwendung der Zusammensetzung gemäß einem oder mehreren der Ansprüche 7 bis 12 als Katalysator.

14. Verfahren zur selektiven und partiellen Oxidation von Kohlenwasserstoffen in Gegenwart von molekularem Sauerstoff und einem Reduktionsmittel, **dadurch gekennzeichnet, dass** man eine Zusammensetzung gemäß einem oder mehren der Ansprüche 7 bis 12 als Katalysator einsetzt.

15. Verfahren gemäß Anspruch 14, **dadurch gekennzeichnet, dass** Propen zu Propenoxid oxidiert wird.

## Claims

1. A process for preparing a composition comprising the steps
1) the preparation of an organic/inorganic hybrid material which contains silicon oxide, 0.1 - 20 mol.% of titanium in oxidic form, with respect to the silicon oxide, and Si-H groups on the external and internal surfaces and is prepared using a sol-gel process, in which silanes of the general formula (IIIa) or (IIIb)
[RₓSiH_{y}(OR')_{4-(x+y})] (IIIa)
[RₓSiH_{y}(Hal)_{4-(x+y)}] (IIIb)
wherein
R and R' are identical or different and are chosen, independently, from the group C₁-C₁₂-alkyl, C₆-C₁₂-aryl, where x = 0, 1, 2, 3 and y = 1, 2, 3 and R may also be H,
are used, and then
2) the organic/inorganic hybrid material is treated once or several times with a soluble gold and/or silver compound.

2. A process according to claim 1, **characterised in that** suitable low molecular weight compounds are mixed in a solvent and then the hydrolysis and/or condensation reaction is initiated by adding water and acid or basic catalysts.

3. A process according to claim 2, **characterised in that** one or more organic acids are used as the catalyst.

4. A process according to one or more of claims 1 to 3, **characterised in that** the composition is conditioned at temperatures in the range 100 to 1000°C in an intermediate or final step.

5. A process according to claim 4, **characterised in that** conditioning is performed at temperatures in the range 200 to 600°C under an inert gas.

6. A process according to claim 1, **characterised in that** the volume of the solution on the support is less than or equal to the pore volume of the titanium-containing organic/inorganic hybrid material with Si-H groups.

7. Compositions obtainable by the process according to claim 1.

8. Compositions according to claim 7, **characterised in that** the organic/inorganic hybrid materials are organically modified glasses and contain terminal and/or bridging organic groups in the network.

9. A composition according to claim 7 and/or 8, **characterised in that** the molar concentration of Si-H groups, with respect to the silicon oxide content, is in the range 0.01 to 100 mol.%.

10. A composition according to one or more of claims 7 to 9, **characterised in that** the organic/inorganic hybrid material contains between 0.1 and 10 mol.%, with respect to silicon oxide, of titanium and optionally other foreign oxides, so called promoters.

11. A composition according to one or more of claims 7 to 10, **characterised in that** it contains between 0.001 and 4 wt.% of gold or between 0.01 and 20 wt.% of silver or a mixture of gold and silver.

12. A composition according to one or more of claims 7 to 11, **characterised in that** the surface has been modified with siliconalkyl(aryl) compounds.

13. Use of the composition in accordance with one or more of claims 7 to 12 as a catalyst.

14. A process for the selective and partial oxidation of hydrocarbons in the presence of molecular oxygen and a reducing agent, **characterised in that** a composition in accordance with one or more of claims 7 to 12 is used as catalyst.

15. A process according to claim 14, **characterised in that** propene is oxidised to propene oxide.

## Revendications

1. Procédé de préparation d'une composition comprenant les étapes suivantes
1) préparation d'une matière hybride organique-inorganique qui contient de l'oxyde de silicium, 0,1 à 20 % en mole de titane sous forme oxydique, par rapport à l'oxyde de silicium, et des groupes Si-H sur les surfaces externe et interne et est préparée par l'intermédiaire d'un procédé sol-gel, dans lequel sont utilisés des silanes de formules générales (IIIa) ou (IIIb) :
[RₓSiH_{y}(OR')_{6-(x+y)}] (IIIa)
[RₓSiH_{y}(Hal)_{4-(x+y)}] (IIIb)
dans lesquelles
R et R' sont identiques ou différents et sont sélectionnés, indépendamment l'un de l'autre, parmi les radicaux alkyle en C₁-C₁₂, aryle en C₆-C₁₂ avec x = 0, 1, 2, 3 et y = 1, 2, 3 et R' et R peuvent aussi être H et, ensuite
2) un traitement simple ou multiple de la matière hybride organique-inorganique avec un composé soluble d'or et/ou d'argent.

2. Procédé selon la revendication 1, **caractérisé en ce que** des composés adéquats de faible poids moléculaire sont mélangés dans un solvant et la réaction d'hydrolyse et/ou de condensation est ensuite amorcée par addition d'eau et de catalyseurs acides ou basiques.

3. Procédé selon la revendication 2, **caractérisé en ce qu'**un ou plusieurs acides organiques sont utilisés comme catalyseur.

4. Procédé selon l'une ou plusieurs des revendications 1 à 3, **caractérisé en ce que** la composition est traitée thermiquement dans une étape intermédiaire ou finale à des températures de l'ordre de 100 à 1000 °C.

5. Procédé selon la revendication 4, **caractérisé en ce que** le traitement thermique est réalisé à des températures de l'ordre de 200 à 600 °C sous gaz inerte.

6. Procédé selon la revendication 1, **caractérisé en ce que** le volume de la solution sur le support est inférieur ou égal au volume des pores de la matière hybride organique-inorganique contenant du titane avec des groupes Si-H.

7. Compositions obtenues par le procédé selon la revendication 1.

8. Compositions selon la revendication 7, **caractérisées en ce que** les matières hybrides organiques-inorganiques sont des verres organiquement modifiés et contiennent des groupes organiques terminaux et/ou de pont dans le réseau.

9. Composition selon l'une des revendications 7 et/ou 8, **caractérisée en ce que** la concentration molaire des groupes Si-H par rapport à la teneur en oxyde de silicium se situe dans une plage de 0,01 à 100 % en mole.

10. Composition selon l'une ou plusieurs des revendications 7 à 9, **caractérisée en ce que** la matière hybride organique-inorganique par rapport à l'oxyde de silicium contient entre 0,1 et 10 % en mole de titane ainsi qu'éventuellement d'autres oxydes étrangers, que l'on appelle des promoteurs.

11. Composition selon l'une ou plusieurs des revendications 7 à 10, **caractérisée en ce qu'**il contient entre 0,001 et 4 % en poids d'or ou entre 0,01 et 20 % en poids d'argent ou un mélange d'or et d'argent.

12. Composition selon l'une ou plusieurs des revendications 7 à 11, **caractérisée en ce que** sa surface a été modifiée avec des composés alkyl(arylés) de silicium.

13. Mise en oeuvre de la composition selon l'une ou plusieurs des revendications 7 à 12 comme catalyseur.

14. Procédé d'oxydation partielle et sélective d'hydrocarbures en présence d'oxygène moléculaire et d'un réducteur, **caractérisé en ce que** l'on utilise une composition selon l'une ou plusieurs des revendications 7 à 12 comme catalyseur.

15. Procédé selon la revendication 14, **caractérisé en ce que** du propène est oxydé en oxyde de propène.
